# EUROPEAN PATENT APPLICATION

(11) **EP 3 967 332 A2**
(43) Date of publication of application: **16.03.2022**
(21) Application number: 21020453.3
(22) Date of filing: 08.09.2021
(51) Int. Cl.: A61L 2/10, A61L 2/18, A61L 2/22, A61L 2/20, B60H 3/00

(54) **SYSTEM FOR THE SANITIZATION OF THE INTERIOR OF A VEHICLE AND METHOD FOR SANITIZATION THEREOF**

(30) Priority: 10.09.2020 IT 202000021457
(71) Applicant: Bellitteri, Davide Benedetto, 91025 Marsala (TP) (IT)
(72) Inventor: Bellitteri, Davide Benedetto, 91025 Marsala (TP) (IT)
(74) Representative: Spagnolo, Chiara

(57) **Abstract**

System for the sanitization of the interior of the vehicle and method for the sanitization thereof, which employs means which emit ultraviolet C rays; means which generate ozone emitting an airflow for the sanitization cycle; said means being of the type suitable for carrying out the internal sanitization of the interior of said vehicle, which system including the following, integrated in the vehicle
- means which emit UVC germicidal rays;
- means which generate ozone emitting an airflow for the treatment cycle;
- means suitable for nebulizing a solution of disinfectant liquid inside the interior of said vehicle;
said means which, being incorporated into the vehicle, are driven and coordinated by means of a common control unit, which at least activates one of said means for the execution of a sanitization cycle of the interior of the vehicle, followed by at least a second one, and then it provides for the ventilation, as well as the possible dehumidification, of the interior of said vehicle.

## Description

The present invention relates to a system and a method for sanitizing the interiors of vehicles.

In this difficult period, people often feel powerless in front of the advancement of the pandemic, so that it is important to exercise meticulous precautions through all means, aimed at reasonably eradicating at least part of pathogenic agents (mostly bacteria and viruses) which, in addition to the problem of one's own health, can cause the arrest or slowdown for example of production chains, thus compromising the economy until the collapse of the entrepreneurial system.

In this context, it is necessary to make use of important sanitization plans, for example of environments, in order to protect the common areas which are the most exposed to contamination as they are frequented by several people of various origins, identifying as the main aim the sanitization of the environments, such as, for example, the interiors of each vehicle by exploiting some chemical processes which allow the reduction of bacteria, viruses, fungi and other organic agents with reasonable and good effectiveness.

### DOMAIN OF THE INVENTION

The innovation has particular, though not exclusive, application in the field of the solutions suitable for the sanitization of any means of transport which is directly supplied by the car manufacturer or also, integrated, as an aftermarket accessory, in relation to the means of transport already on the market in order to ensure a healthy environment in the interior.

Thus, in general, it can be agreed that the following innovation can be applied to any vehicle used for the transport of things and/or people such as, by way of example, private cars, official cars such as state cars, taxis, ambulances, buses, trains, trolley buses, trams, commercial vehicles for the transport of things, such as food and non-food items, also armored means of transport, hearses, articulated lorries, generic trailer trucks, trailers, articulated vehicles, not excluding boats, ships in general, and still more, such as housing modules.

In this case, it is known that, before the pandemic, the public having to move from one place to another, for tourism or work, entered the interiors of vehicles mostly without worrying about coming into contact with surfaces that, in all probability, were somehow already contaminated by more or less aggressive pathogenic agents. Today, it can be said that this is no longer the case, this due to the fact that the public, people, are progressively more and more worried about coming into contact, mostly incidentally, with generic surfaces in various ways and previously contaminated due to various reasons, which surfaces, despite the precautions taken, have the potential to transmit infectious diseases by direct contact, translation or by air.

The consequence, in one of the possible cases, is that not only clothes are, in turn, contaminated by coming into contact with surfaces involved by an indiscriminate number of people, but above all the exposed parts of the body, of which the classic example are hands, arms (elbows), sometimes legs and mostly rarely the back.

The aforementioned inconvenience, which is frequent in terms of probability, can be solved by wearing suitable disposable garments and using special cleaning and sanitizing precautions for the exposed parts of the body, for example by sanitizing the hands whenever they touch a surface potentially already touched by third parties. Nevertheless, although the prescribed rules of hygiene and sanitization of the environments are also adopted, which are imposed by regulations, prescriptions and ultimately the common sense of each individual, these actions can be not always easily exercised, either due to the lack or insufficient use of sanitization services, or because there are no protective devices, in other words, to stay in your home is one thing, to make use of environments is another, such as, for example, the interior of the car or other means of transport, relative to which there is no knowledge of the state of health of those who have previously used them and who theoretically could carry an indefinite number of germs.

Due to the current known pandemic situations, it can be said that if one thinks in terms of possible contamination, it therefore happens, as food for thought, that the perception of serious danger is not so far from reality, so that feeling somehow helpless, where neither masks nor gloves certainly allow to safeguard the clothes or the body which thus remain particularly exposed, almost gives rise to the certainty, the fear, of being inadvertently infected.

It is widely known, in the sanitization sector, that ultraviolet (UV) lamps with a wavelength lower than 320nm, or with a spectral zone mostly between 200nm and 280nm, or still UV-C, also in LED version, through the photolytic function have a germicidal effect, acting on the DNA of bacteria, molds, leavens and viruses, preventing their reproduction.

As a consequence, in recent periods, there has been a rapid increase of solutions which, to sterilize objects or environments, have essentially intended to use UV-C lamps which, once activated by means of a control and command logic unit, irradiate said objects or environments according to a treatment cycle which includes one or more phases.

In the same way, it can be agreed that another means of sanitization, commonly used in many sectors, not excluding environments, derives from the use of ozone. Among all the existing disinfectants, ozone is considered to be the best for eliminating germs and bacteria. Its very high oxidizing power makes it an effective sanitizer and deodorant, even more effective than traditional chemical detergents. It is natural, ecological and economical, and also thanks to its properties, ozone sanitizes, oxygenates and regenerates the air we breathe besides disinfecting the water we use. If used intelligently, it eliminates bacteria, molds, fungi, leavens, pollens and mites and it inactivates viruses, and it is a powerful ally against allergies, asthma and infections, because it has the power to cut down the microbial charge present in the air and on surfaces.

### PRIOR ART

In the sector of vehicle sanitization, for example, the use of UV-C lamps is known. KR20190094754 (Hang) describes a portable air purifier for a vehicle, provided with a UV LED sterilizer and dust collector and which uses sunlight as a power source. The portable air purifier for a vehicle comprises: a UV LED sterilization module formed on a side surface of a case cover; solar cell panels formed on the upper surface of the case cover and on each side surface of a case; a dust collection module obtained inside the case; a filter; a part of electrical energy storage; a control part; a driving part; and an air suction fan.

KR20200080058 (Cure) teaches that an automated cleaning device for washing the exterior of a vehicle is widely used as a device for maintaining the cleanliness of a vehicle, but it only cleans the exterior of the vehicle by spraying water and cleaning liquid, liquid water and cleaning liquid. Therefore, there is the problem of a limitation in the sterilization and disinfection, sanitization, of the vehicle inside. The relative state of the art mentions the fact that patent registration no. 10-0999370 describes a vehicle sterilization and disinfection system, but the prior art cannot determine whether vehicle sterilization and disinfection are performed in a correct process by the vehicle sterilization and disinfection system. Depending on the worker using the sterilization and disinfection system, it is difficult to maintain the quality and level constant of the vehicle sterilization and disinfection service, so there is a problem that consumers cannot trust the service.

The invention proposed in this cited document was conceived to solve the above problems, and the problem to be solved in the present invention is a complex sterilization unit for sterilizing a vehicle mat, a UV irradiation unit for sterilizing a vehicle seat and a sterilizing air conditioner also to sterilize a vehicle's air conditioner. It provides a vehicle sterilization management system and vehicle sterilization management method which can handle whether the vehicle sterilization process is carried out correctly by comparing mat sterilization completion information, coating sterilization completion information and the air conditioner sterilization completion information transmitted by each unit with the sterilization information standard. The vehicle sterilization management system as described to solve the above problems is a complex sterilization unit which sterilizes a vehicle mat by irradiating ultraviolet rays and transmits the mat sterilization completion information including information on the time to sterilize the vehicle mat. A UV irradiation unit which irradiates ultraviolet rays to sterilize the vehicle seat and transmits the sheet sterilization completion information, including information on the time to sterilize the vehicle seat; An air conditioning sterilization unit which sprays a chemical solution in nebulized form to sterilize the vehicle's air conditioning unit and transmits the air conditioning sterilization completion information, including information on the time to sterilize the vehicle's air conditioning unit; And receive the mat sterilization completion information, seat coating sterilization completion information and the air conditioner sterilization completion information transmitted by the complex sterilization unit, by the UV irradiation unit and by the air conditioner sterilization unit and mat sterilization completion information received, sterilization completion information and air conditioning sterilization completion information. It is characterized in that it includes a control unit for comparing information and standard sterilization information to generate and transmit vehicle sterilization completion information.

CN207565281 (Univ) describes the use of an air purifier on the vehicle based on photocatalysis, including the separator case, the bottom of the frame is provided with the air inlet window, and the side part is provided with the air outlet and there are primary effect filter layers, activated carbon adsorption layer, nanometric photocatalysis layer in the inner opening of the air inlet mouth in the correct order and nanometric photocatalysis layer on both sides are provided with ultraviolet lamp and the inside of both sides of the air inlet mouth are still provided with generator of anions and respectively of ozone. The photocatalysis-based air purifier on the vehicle is used previously through the primary effect of the dust particle filter layer, again through the activated carbon absorption layer which absorbs the peculiar smell and fine particles, again through the disinfection purifications of the nanometric photocatalysis layer, simultaneously cooperating for the disinfection through the disinfection function of the anion generator and the ozone generator.

CN207565281 (Univ) refers to the field of air purification and describes a method of air purification for interior environments and cars. The method is characterized in that it includes the basic purification, secondary purification, mechanical purification and final treatment steps in sequence and it includes the following specific operational steps: a. basic purification: maintaining the cleanliness and dryness of an internal coated and internal surface and uniform spraying of a photocatalyst anywhere on the coated surface; b. secondary purification: uniformly spraying a formaldehyde scavenger everywhere inside and in the interior of a car; c. mechanical purification: placing an ozone machine inside and in the interior of the car, where the processing time of the ozone machine is 15-20 hours; and d. final treatment: opening the windows for ventilation, which allows to disperse the internal air, uniformly spraying a lytic enzyme preparation, and then spraying the formaldehyde scavenger again. The method has the beneficial effects of sufficient dilution and release, repurification, cost reduction and efficiency improvement; and through re-dilution and repurification, the noxious gas is reduced to harmless salt, inhibiting its return.

It is therefore reasonable to consider as known on the whole:
- the use of UV lamps with germicidal action and the use or general purpose of them to sanitize environments, airplanes and surfaces;
- the use of multiple UV lamps, also mounted in correspondence with the surfaces of the interior, for example on the wall or ceiling for systematic sanitization of the environment, to proceed with a controlled sanitization cycle;
- the use of UV lamps in germicidal equipment to be placed inside the vehicle;
- the use of ozone generators to disperse the disinfectant flow inside the interior of the vehicle and then to air or to ventilate the vehicle with external air before its use;
- in literature, also the use of solutions based on ethyl alcohol at 62-71% V/V, alternatively hydrogen peroxide at 0.5% or sodium hypochlorite at 0.1% active chlorine for at least 1 minute, have demonstrated good efficacy as a surface disinfectant.

### DRAWBACKS

Following tests, including laboratory tests carried out in relation to some pre-existing solutions, it was possible to highlight the fact that the final result of the sanitization treatment was not found in a homogeneous, uniform way for each one of the surfaces of the interior of a vehicle. In other words, the values found, for example, in relation to the bacterial mass at the end of the process and inside the interior, were significantly different in relation to each affected surface and such as to detect a reasonable non-uniformity of the treatment between them, a circumstance that, in the opinion of the drafter, has effectively prevented, to date, the optimization of said solutions, above all for effectively sanitizing the interiors of the vehicles.

Furthermore, in the field of the solutions mentioned above, solutions are not found or suggested designed to be integrated and interacting with each other, particularly to be used in a rational and effective way.

An essential aim of the present invention is also to avoid the aforementioned drawbacks.

### BRIEF DESCRIPTION OF THE INVENTION

This and other aims are reached with the present innovation according to the characteristics as in the attached claims, solving the problems set out by means of a system for the sanitization of the interior of the vehicle and method for the sanitization thereof, which employs means which emit ultraviolet C rays; means which generate ozone emitting an airflow for the sanitization cycle; said means being of the type suitable for carrying out the internal sanitization of the interior of said vehicle, which system including the following, integrated into the vehicle
- means which emit UVC germicidal rays;
- means which generate ozone emitting an airflow for the treatment cycle;
- means suitable for nebulizing a solution of disinfectant liquid inside the interior of said vehicle;
   said means which, being incorporated into the vehicle, are driven and coordinated by means of a common control unit, which at least activates one of said means for the execution of a sanitization cycle of the interior of the vehicle, followed by at least a second one, and then it provides for the ventilation, as well as the possible dehumidification, of the interior of said vehicle.

### ADVANTAGES

In this way, through the considerable creative contribution the effect of which constitutes an immediate technical progress, some aims and advantages are achieved.

In principle, it is possible to achieve the optimization as well as complete sanitization of the surfaces of the interiors of vehicles and also intervening on the external surfaces which may come most likely into contact with potentially contaminated subjects, such as the opening handles of the doors for entering the interior.

Ultimately, therefore, greater efficiency and accuracy, at the same time, combining the need not to modify or impact aesthetically, therefore a functional complement particularly suitable in the automotive sector, practical to be used in an aesthetically pleasing environment, even ergonomic, taking into account the spaces. The object of the invention, indeed, appears mostly dedicated to the rational use inside the interiors and in any case, mainly intended for vehicles dedicated to the transport of people for short periods and journeys, such as taxis.

Another advantage may consist in making it possible for the public to use a single sanitization system, which is configured in such a way as to achieve at least three advantages for the user, namely:
a) it is a valid system which sanitizes surfaces;
b)it performs a programmable function suitable for preventing the aggression or proliferation of contaminants during the period of non-use of the vehicle;
c) it is also an effective system for sanitizing the ambient air in the interior.

In particular, it must be said that the activity of irradiation by means of germicidal ultraviolet lamps, also of the air recirculation flow in a seat limited by the restricted volume of the interior, allows an effective treatment of sanitization, sterilization, at the same time affecting the surface of the interior with punctuality and uniformly. Through the logic control unit, modulating the irradiation of the germicidal lamps, together with the air circulation and/or the ozonization and/or nebulization activity of a sodium chloride solution, in relation to a predetermined period, for each phase of the procedure in a cycle, whether it is of the "fast" or "complete" type, allows to further increase the efficacy of the sanitization treatment.

A further advantageous aspect derives from the versatility of the system, which by operating with the logic unit offers the possibility for the user to alternatively choose at least one of the aforementioned three main functions, based on the degree of sanitization to be achieved.

These, but also other advantages or aims will appear from the following detailed description of at least one preferential embodiment solution with the aid of the attached schematic drawings whose execution details are not to be considered limitative but only illustrative.

### CONTENT OF THE DRAWINGS

**Figure 1** is a front view of the motorcar, which integrates the system to sanitize the interior represented for the execution of the complete sanitization cycle;
**Figure 2** is a view of the motorcar shown in Fig.1, taken from the rear side;
**Figure 3** is a view from above of the motorcar shown in Fig.1;
**Figure 4, 4a** **e** **4b****,** is a side view and a view of details of the motorcar shown in Fig. 1;
**Figure 5** is a front view of the motorcar shown in Fig. 1, which integrates the system to sanitize the interior represented for the execution of the fast sanitization cycle;
**Figure 6** is a view from the rear side of the motorcar shown in Fig. 5;
**Figure 7** is a view from above of the motorcar shown in Fig. 5;
**Figure 8, 8a** **e** **8b****,** is a side view and a view of details of the motorcar shown in Fig. 5;

### PRACTICAL DESCRIPTION OF AN EMBODIMENT OF THE INVENTION

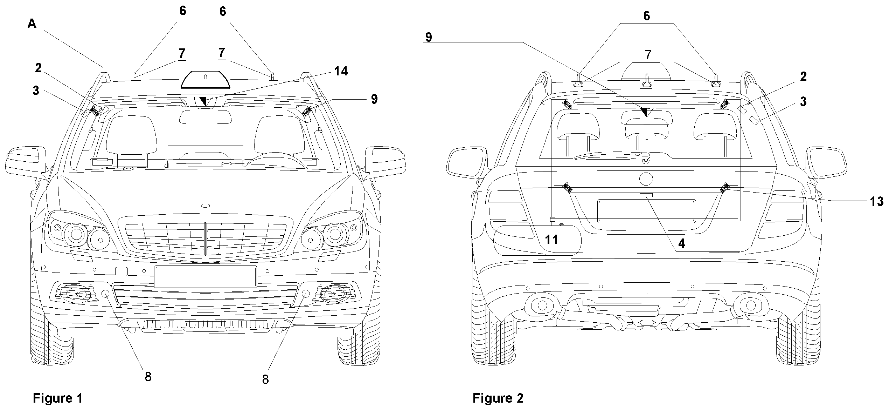

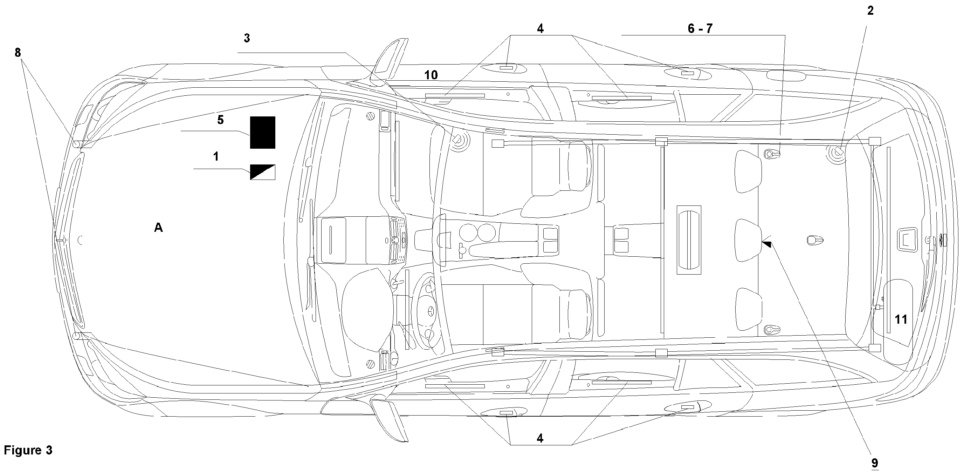

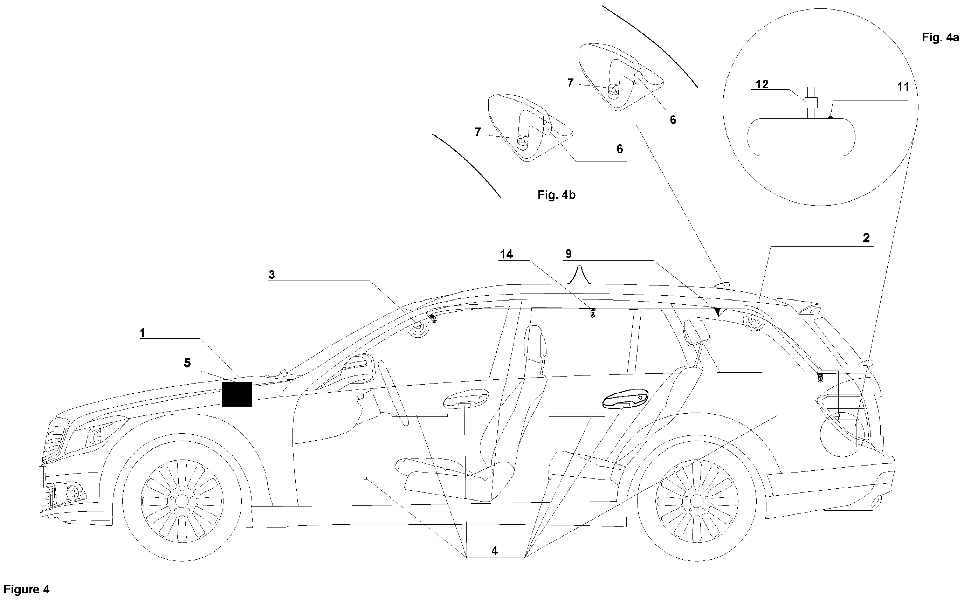

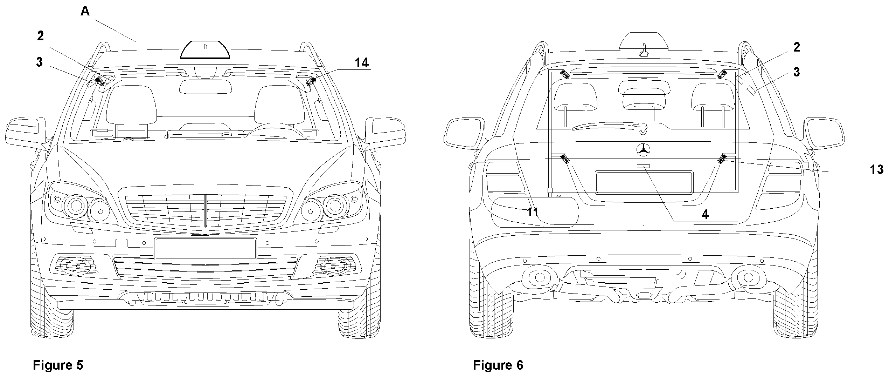

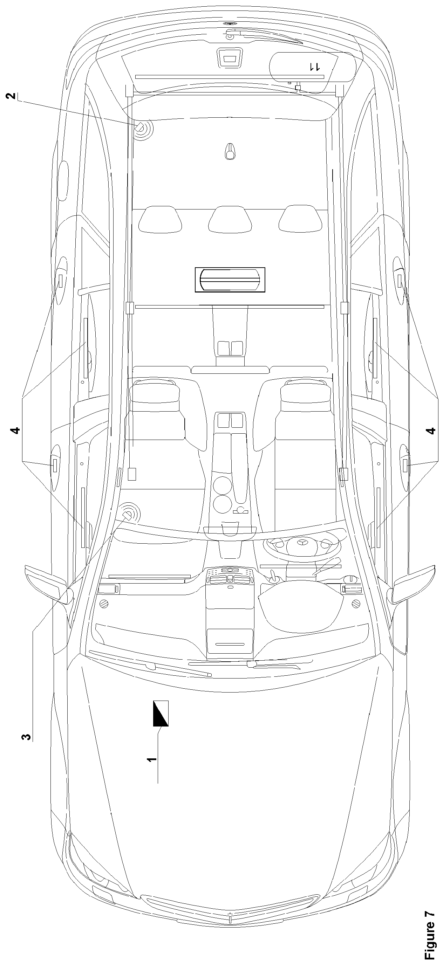

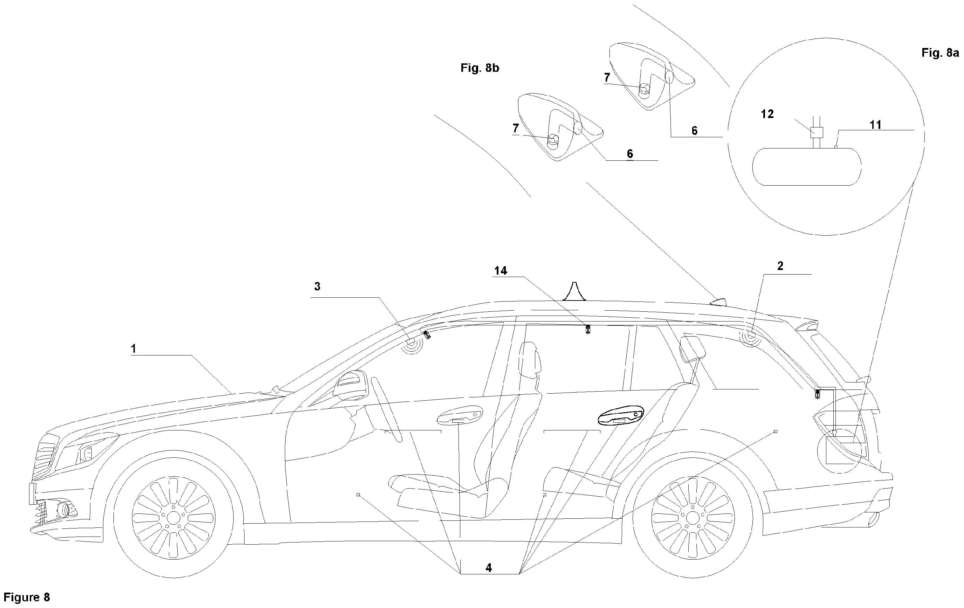

A common car (A) was taken as a case study. For the sanitization of the interior of said vehicle (A), two procedures are identified precisely named:
**Complete sanitization cycle,** the sanitization procedure of which requires longer times (but it is a process more effective than the fast phase); see Figure 1 - Complete phase, Figure 2 - Complete phase, Figure 3 - Complete phase, Figure 4 - Complete phase.
**Fast sanitization cycle,** the sanitization procedure of which requires reduced times (approx. 1-2 minutes) but it is a process less effective than the complete phase; see Figure 5 - Fast phase, Figure 6 - Fast phase, Figure 7 - Fast phase, Figure 8 - Fast phase.

The possible combination of the two cycles, complete and fast, allows to ensure a healthy environment in the interior for the whole day (24 hours).

In particular, the complete cycle can be started, for example, when you return home in the evening or park your vehicle in a garage. In this procedure, a sanitization process will be activated at different times, which also includes the phase of ozonization and nebulization of a sodium chloride solution.

As for the fast cycle, it can be started at any time of the day at the discretion of the owner of the vehicle or public means of transport.

In order to check whether sanitization has been carried out correctly in the interior of the vehicle (A), it will be necessary to provide an indicator having the shape of a traffic light (green/red) on the display of the control board inside the interior of the vehicle (A). If said display shows the green signal the sanitization has been carried out correctly, if the indicator shows the red colour, vice versa, it will indicate that the sanitization has not been carried out correctly, while the turning on of the yellow colour, in the display, may possibly indicate that the sanitization procedure is in progress. Alternatively, the same red colour lighting of the indicator may indicate that the sanitization procedure is in progress.

Each time the sanitization procedure is completed, an acoustic signal will be emitted which will indicate the end of the process.

The sanitization process (complete or fast cycle) will have to be started by means of the starting keys of the motor vehicle or by means of dedicated Application which can be downloaded on remote devices.

Indication of the operating phases 'Complete sanitization cycle' (Figs. 1-4).

The procedure to be driven and coordinated by the logic unit of the control unit (1), Figure 3, will be as follows:
- Presence detectors (2) e (3); (Safety condition)
- Verification of the closure of the doors (10); (Safety condition)
- the lighting on of the UVC germicidal lamps (4); (for example of the LED strip type)
- Activation of the sanitization function which provides at least the irradiation of the interior of the vehicle (A) by means of said UVC LEDs (4), said UVC LEDs (4) being also placeable in correspondence with the external handles of the entrance doors of said motor vehicle (A);
- Activation of ozonizer (5);
- Opening of ozone emission mouths (6) and opening of aspirators (7) obtained on the vehicle roof (A);
- Opening of "clean air" entry mouths and filter (8) and opening of aspirators;
- Ozone detector (9);
- Closure of mouths and closure of aspirators (6) and (7);
- Turning off of UVC LEDs (4).

With the activation of the UVC LEDs (4) which are placed in this case in correspondence with the internal panels of the doors (10) of said vehicle (A) a very effective germicidal action is achieved even though prolonged exposure to the human body can lead to burns and inflammation of the cornea, while the activation of the ozonizer (5) is an effective action to sanitize the air and the environments even though prolonged exposure to the human body may cause irritation to the respiratory tract.

To protect the person, before these procedures are started by the control unit (1), the following conditions must be met:
Absence of people inside the interior using said presence sensors (2, 3);
Verification that the doors (10) are closed and that the windows are raised.

In the only case in which the presence sensors (2, 3) detect no movements, the doors (10) are closed and the windows are raised, the procedure is activated consisting of:
- Turning on of UVC LEDs (4);
- Activation of the sanitization process;
- Activation of the ozonization process.

### Turning on of UVC LEDs

The UVC LEDs (4), arranged inside and outside the interior (Fig. 3), will remain turned on for the entire working cycle (both sanitization and ozonization).

### Activation of the sanitization phase

Simultaneously with the turning on of the UVC LEDs (4), the sanitization process will be activated. A 30-liter container (11) will be placed in the boot where water mixed with a chlorine dioxide solution will be allocated (the latter will act as a disinfectant - non-flammable). By means of a pump (12), placed in the proximity of the container, the nebulized liquid will be introduced on two paths consisting on the whole of nozzles (13, 14) each one arranged (as per layout) in the following way:
Path 1 which connects:
   A first nozzle (13) which will nebulize the right side boot;
   2 nozzles (14) which will nebulize the right rear seat and right front seat.
Path 2 which connects:
   A second nozzle (13) which will nebulize the left side boot;
   2 nozzles (14) which will nebulize the left rear seat and left front seat.

Said path will be made with small rubber tubes arranged, for example, between the roof of the car and the upper lining where the nozzles (14) will be located. It is preferred to place the nozzles (14) at 45 ° in such a way that the nebulized liquid does not go onto the window panes.

### Activation of the ozonization process

Upon completion of the sanitization process, the ozonization process will be activated. Said ozonizer (5) will be placed inside the bonnet of the car in the proximity of the control unit (1). The function of the ozonizer will be to convert all the oxygen present in the motor vehicle into ozone. Upon completion of the procedure, the control unit (1) will have to deactivate the ozonizer and activate simultaneously:
Opening of the 4 mouths of which:
   2 of them placed (6) inside the fins on the roof of the car;
   2 of them placed (8) in the lower parts of the motorcar connected to two filters;
Starting of the 4 aspirators (7) of which:
   2 of them placed inside the fins on the roof of the vehicle;
   2 of them placed in the lower part of the motorcar;
   the third and fourth mouth together with the filters and the aspirators will have the task to take clean air from the vehicle (where the car or the means of transport is parked) and to facilitate the outflow of ozone from the other two mouths placed in the upper parts. This procedure will continue until no more ozone is present in the motorcar detected by means of an ozone detector. Upon completion of the procedure the 4 aspirators will be turned off and the 4 mouths will be closed. Afterwards the UVC LEDs (4) will be turned off.

### Fast sanitization cycle

The procedure to be carried out by the control unit (see Figs 5-8) will be the following:
- Presence detectors (2) and (3); (Safety condition)
- Verification of the closure of the doors (10); (Safety condition)
- Turning on of UVC LEDs (4);
- Activation of the sanitizer;
- Turning off of UVC LEDs (4).

The activation of UVC LEDs (4) generates a very effective germicidal action even though, as mentioned, prolonged exposure to the human body can cause burns and inflammation of the cornea. To protect the person, before this procedure is started by the control unit (1), the following conditions must be met:
- Absence of people inside the interior using 2 presence sensors (2, 3);
   - Verification that the doors (10) are closed and that the windows are raised;

In the only case in which the presence sensors (2, 3) detect no movements, the doors (10) are closed and the windows are raised, the procedure is activated consisting of:
- Turning on of UVC LEDs (4);
- Activation of the sanitization process.

### Turning on of UVC LEDs

The UVC LEDs (4), arranged inside and outside the interior, will remain turned on for the entire working cycle (sanitization).

### Activation of the sanitization phase

Simultaneously with the turning on of the UVC LEDs (4), the sanitization process will be activated. In the boot the 30-liter container (11) is intended to contain water mixed with a chlorine dioxide solution (the latter will act as a disinfectant - non-flammable). By means of the pump (12), placed in the proximity of the container, the nebulized liquid will be introduced on two paths consisting of nozzles (13, 14) arranged (as per layout) in the following way and as already described:
Path 1 which connects:
   A nozzle (13) which will nebulize the right side boot;
   2 nozzles (14) which will nebulize the right rear seat and right front seat.
Path 2 which connects:
   A nozzle (13) which will nebulize the left side boot;
   2 nozzles (14) which will nebulize the left rear seat and left front seat.

Said path will be possibly made with small rubber tubes which thus, as already described, are arranged between the roof of the car and the upper lining where the nozzles (14) will be located. It is preferred to place the nozzles (14) at 45 ° in such a way that the nebulized liquid does not go onto the window panes.

Afterwards the UVC LEDs (4) will be turned off.

### Functionality of the App dedicated to the management and remote coordination of the functions and of the cycles of sanitization provided for in the present invention

The application which will have to be implemented for all mobile devices (Android and iOS) will have to allow, at any time, the possibility to opt for both the complete as well as fast cycle sanitization (besides the possibility to program them at a given moment or time interval). Furthermore, independently of the choice of one phase or the other, the application will have to communicate, at the end of the cycle, whether the sanitization has correctly taken place or not, by emitting an acoustic signal (beep) and possibly together with a signalling through signals or light and/or messaging icons.

The system will automatically send the occurred sanitization confirmation to an already memorized e-mail address.

By accessing the App, the system will have to recognize (by means of GPS) the motor vehicles (in the proximity) provided with the sanitization system, and indicate for each motor vehicle when the last fast and complete sanitization took place. In this case, for example for taxis, the application provides an indication of whether the called taxi has been properly sanitized, so that if it is not, it can exclude the call.

Having identified the path to follow, the application will also have to indicate all atmospheric information (temperature and weather forecasts).

From the settings menu of the App it will be possible to verify:
- the level of the container (11) of water (so that if it falls below a given value the App will communicate this);
- when to introduce the chlorine dioxide;
- the conditions of the nozzles (13, 14);
- the functioning status of the UVC LEDs (4);
- the functioning of presence sensors (2, 3);
- the functioning status of the ozonizer (5).

In a possible preferential solution, in the case of use of electric vehicles, the sanitization function can also be started in a completely automatic way, without using the application every time said vehicle is connected to the charging post for recharging the electric batteries, making sure that the control unit (1) recognizes the connection of the charging socket, as an input for starting the sanitization cycle.

## Claims

1. System for the sanitization of the interior of the vehicle and method for the sanitization thereof, which employs means (4) which emit ultraviolet C rays; means (5) which generate ozone emitting an airflow for the sanitization cycle; said means (4, 5) of the type suitable for carrying out the internal sanitization of the interior of said vehicle (A), **characterised in that** it includes:
- means (4) which emit UVC germicidal rays;
- means (5) which generate ozone emitting an airflow for the treatment cycle;
- means (11, 12, 13, 14) suitable for nebulizing a solution of disinfectant liquid inside the interior of said vehicle (10);
said means (4, 5, 11, 12, 13, 14), which, being incorporated into the vehicle (10), are driven and coordinated by means of a control unit (1), which at least activates, in sequential order, a first one followed by at least a second one of said means (4, 5, 11, 12, 13, 14) for the execution of a sanitization cycle of the interior of the vehicle (10).

2. System for the sanitization of the interior of the vehicle and method for the sanitization thereof, according to claim 1., **characterised in that** the means (4) which emit UVC germicidal rays are placed at least in correspondence with the internal panel of the door (10) and with the external handles; the means (5) which generate ozone are located in the proximity of the motor bonnet adjacent to the control unit (1), and are connected to mouths and aspirators (6, 7, 8) in such a way as to allow the emission of an ozonized airflow inside the interior and to expel it; the means (4, 5, 11, 12, 13, 14) for the execution of a sanitization cycle of the interior of the vehicle (10), including a container (11) intended to contain water mixed with a chlorine dioxide solution, from which, by means of the pump (12), placed in the proximity of the container (11) the nebulized liquid is introduced on two paths composed of nozzles (13, 14) arranged in the boot and in the interior, in such a way as to involve the zone relative to the rear and front seats.

3. System for the sanitization of the interior of the vehicle and method for the sanitization thereof, according to claim 1. and 2., **characterised in that** in the interior of said vehicle (10) there are people presence detectors (2, 3), said detectors communicating with the control unit (1) in such a way as to interrupt or not to activate a sanitization cycle when people are present inside said interior of the vehicle (10).

4. System for the sanitization of the interior of the vehicle and method for the sanitization thereof, according to the previous claims, **characterised in that** on the display of the control board, inside the interior of the vehicle (A) there is an indicator having the shape of (green/red) traffic-light, interfaced with the control unit (1) in such a way that, if said display shows the green signal the sanitization has been carried out correctly, if the indicator shows the red colour, vice versa, it will indicate that the sanitization has not been carried out correctly, while the turning on of the yellow colour, in the display, may possibly indicate that the sanitization procedure is in progress; alternatively the same red colour lighting of the indicator may indicate that the sanitization procedure is in progress; and still in which each time the sanitization procedure is completed, an acoustic signal will be emitted which will indicate the end of the process.

5. System for the sanitization of the interior of the vehicle and method for the sanitization thereof, according to the previous claims, **characterised in that** the sanitization process is started by means of the starting keys of the motor vehicle or by means of dedicated Application which can be downloaded on remote devices, being interfaced with the control unit (1); said Application allowing the possibility to opt for both the complete as well as fast cycle sanitization besides the possibility to program them at a given moment or time interval, and in which independently of the choice of one phase or the other, the application communicates, at the end of the cycle, whether the sanitization has correctly taken place or not, by emitting an acoustic signal and possibly together with a signalling through signals or light and/or messaging icons.

6. System for the sanitization of the interior of the vehicle and method for the sanitization thereof, according to claim 5., **characterised in that** the application automatically sends the occurred sanitization confirmation to an already memorized e-mail address, and in which by means of GPS it recognizes the motor vehicles in the proximity, provided with the sanitization system, and it indicates for each vehicle when the last fast and complete sanitization took place; having identified the path to follow, the application indicates atmospheric information related to the temperature and weather forecasts; and wherein from the settings menu of said application it is possible to verify:
- the level of the container (11) of the water (so that if it falls below a given value the app will communicate this);
- when to introduce the chlorine dioxide;
- the conditions of the nozzles (13, 14);
- the functioning status of the UVC LEDs (4);
- the functioning of presence sensors (2, 3);
- the functioning status of the ozonizer (5).

7. Method for the sanitization of the interior of the vehicle for a sanitization system according to previous claims, **characterised in that** the complete cycle of sanitization driven and coordinated by the logical unit of the control unit (1), provides:
- The preliminary verification of the safety condition by means of said presence detectors (2) and (3);
- The preliminary verification of the closure of the doors (10) and of the windows;
- The lighting on of the UVC germicidal lamps (4);
- Activation of the sanitization function which provides at least the irradiation of the interior of the vehicle (A) by means of said UVC LEDs (4), said UVC LEDs (4) being also placeable in correspondence with the external handles of the entrance doors of said motor vehicle (A);
- Activation of ozonizer (5) and introduction of ozonized airflow into the interior of the vehicle (A);
- Opening of ozone emission mouths (6) and opening of aspirators (7) obtained on the vehicle roof (A);
- Opening of "clean air" entry mouths and filter (8) and opening of aspirators;
- Ozone detector (9);
- Closure of mouths and closure of aspirators (6) and (7);
- Turning off of UVC LEDs (4);
and wherein, for the turning on of UVC LEDs (4), the activation of the sanitization process; and/or the activation of the ozonization process, and/or the activation of the nebulization process of the disinfectant liquid, for the purpose of protecting the person, before these procedures are started by the control unit (1), the following conditions must be met: Absence of people inside the interior employing said presence sensors (2, 3) and verification that the doors (10) are closed and that the windows are raised.

8. Method for the sanitization of the interior of the vehicle for a sanitization system according to claim 7., **characterised in that** in the only case in which the presence sensors (2, 3) detect no movements, the doors (10) are closed and the windows are raised, the procedure is activated of turning on UVC LEDs, arranged inside and outside the interior, and in which simultaneously to the turning on of the UVC LEDs (4) there will be the activation of the sanitization process, and/or the activation of the ozonization process by means of the ozonizer (5), and/or the activation of the nebulization process of the disinfectant liquid.

9. Method for the sanitization of the interior of the vehicle for a sanitization system according to claims 7. and 8., **characterised in that** by means of a pump (12), placed in the proximity of the container (11) the nebulized disinfectant liquid is introduced on two paths composed of nozzles (13, 14) each one arranged according to a first and a second path, said first path connecting a first nozzle (13) which will nebulize the right side boot and two nozzles (14) which will nebulize the right rear seat and right front seat;
said second path connecting a second nozzle (13) which will nebulize the left side boot and two nozzles (14) which will nebulize left rear seat and left front seat,
said first and second path providing small rubber tubes arranged between the roof of the vehicle and the upper lining where the nozzles (14) will be located, which are placed at 45° in such a way that the nebulized liquid does not go onto the window glasses.

10. Method for the sanitization of the interior of the vehicle for a sanitization system according to claims 7. to 9., **characterised in that** upon completion of the sanitization process by means of the UVC germicidal lamps (4), the ozonization process will be activated by means of ozonizer (5) placed inside the bonnet of the vehicle in the proximity of the control unit (1); said function by means of the control unit (1) will have to deactivate the ozonizer (5) and activate simultaneously the opening of the 4 mouths of which:
2 of them placed (6) inside the fins on the roof of the vehicle;
2 of them placed (8) in the lower parts of the motorcar connected to two filters;
Starting of the 4 aspirators (7) of which:
2 of them placed inside the fins on the roof of the vehicle;
2 of them placed in the lower part of the motorcar;
the third and fourth mouth together with the filters and the aspirators having the task to take clean air from the vehicle and facilitating the outflow of ozone from the other two mouths placed in the upper parts; said procedure continuing until no more ozone is present in the motorcar detected by means of an ozone detector and wherein successively the UVC LEDs (4) will be turned off.

11. Method for the sanitization of the interior of the vehicle for a sanitization system according to previous claims, **characterised in that** the sanitization fast cycle, before being started by the control unit (1), verifies at least the following conditions:
- Absence of people inside the interior, using 2 presence sensors (2, 3);
- Verification that the doors (10) are closed and that the windows are raised;
and in the only case in which the presence sensors (2, 3) detect no movements, the doors (10) are closed and the windows raised the procedure is activated consisting of:
- Turning on of UVC LEDs (4), arranged inside and outside the interior, which remain turned on for the whole working cycle;
- Activation of the sanitization process, which requires simultaneously the turning on of the UVC LEDs (4) and the activation of the nebulization phase of the disinfectant liquid taken from the container (11) by means of the pump (12), introducing the nebulized liquid on two paths composed of nozzles (13, 14), of which a first path connecting a nozzle (13) which will nebulize the right side boot and two nozzles (14) which will nebulize right rear seat and right front seat; and a second path connecting a nozzle (13) which provides the nebulization of the left side boot and two nozzles (14) which act for the area relative to the left rear seat and left front seat.
